# EUROPÄISCHE PATENTSCHRIFT

(11) **EP 1 874 762 B1**
(45) Veröffentlichungstag und Bekanntmachung des Hinweises auf die Patenterteilung: **14.01.2009**
(21) Anmeldenummer: 06724257.8
(22) Anmeldetag: 12.04.2006
(51) Int. Cl.: C07D 405/06

(54) **VERFAHREN ZUR HERSTELLUNG VON 5-(4-[4-(5-CYANO-3-INDOLYL)-BUTYL]-1-PIPERAZINYL)-BENZOFURAN-2-CARBOXAMID**
METHOD FOR THE PRODUCTION OF 5-(4-[4-(5-CYANO-3-INDOLYL)-BUTYL]-1-PIPERAZINYL)-BENZOFURAN-2-CARBOXAMIDE
PROCEDE DE PRODUCTION DE 5-(4-[4-(5-CYANO-3-INDOLYL)-BUTYL]-1-PIPERAZINYL)-BENZOFURAN-2-CARBOXAMIDE

(30) Priorität: 26.04.2005 DE 102005019670
(43) Veröffentlichungstag der Anmeldung: 09.01.2008
(73) Patentinhaber: Merck Patent GmbH, 64293 Darmstadt (DE)
(72) Erfinder: BATHE, Andreas, 64283 Darmstadt (DE)
(86) Internationale Anmeldenummer: PCT/EP2006/003344
(87) Internationale Veröffentlichungsnummer: WO 2006/114202

(56) Entgegenhaltungen:
- HEINRICH ET AL: "Synthesis and Structure-Activity Relationship in a Class of Indolebutylpiperazines as Dual 5-HT1A Receptor Agonists and Serotonin Reuptake Inhibitors" JOURNAL OF MEDICINAL CHEMISTRY, Bd. 47, Nr. 19, 2004, Seiten 4684-4692, XP002388367 in der Anmeldung erwähnt

## Beschreibung

Gegenstand der Erfindung ist ein Verfahren zur Herstellung von 5-(4-[4-(5-Cyano-3-indolyl)-butyl]-1-piperazinyl)-benzofuran-2-carboxamid sowie dessen physiologisch unbedenklichen Salze durch chemoselektive, metallkatalysierte Kupplung der Vorstufe 3-(4-Piperazin-1-yl-butyl)-indol-5-carbonitril mit halogenierten Benzofuran-2-Carboxamid-Derivaten.

Gegenstand der Erfindung ist weiterhin ein Verfahren zur Herstellung von 5-(4-[4-(5-Cyano-3-indolyl)-butyl]-1-piperazinyl)-benzofuran-2-carboxamid sowie dessen physiologisch unbedenklichen Salze durch reduktive Aminierung von 3-(4-Oxobutyl)-1H-indol-5-carbonitril mit 5-Piperazinyl-benzofuran-2-carboxamid.

Der bisherige Zugang basiert auf der Kupplung eines aktivierten 5-Cyano-3-Butylindols mit 5-Piperazinbenzofuran-Derivaten wie z.B. aus J. Med. Chem. (2004), 47(19), 4684-4692, Heinrich, T.; Boettcher, H.; Gericke, R.; Bartoszyk, G. D.; Anzali, S.; Seyfried, C. A.; Greiner, H. E.; van Amsterdam, C. und J. Med. Chem. 2004,47,4677-4683, Heinrich, T, Böttcher, H. Bartoszyk, G. D. Greiner, H. E. Seyfried, C. A., van Amsterdam, C. und dort zitierter Literatur bekannt.

Überraschenderweise ergaben nun Untersuchungen im Rahmen der Synthese von Arzneimitteln, welche z.B. in der EP 0 648 767 beschrieben sind, dass 5-(4-[4-(5-Cyano-3-indolyl)-butyl]-1-piperazinyl)-benzofuran-2-carboxamid im Vergleich zum Stand der Technik in wenigstens vergleichbarer oder höherer Gesamtausbeute erhalten werden kann, wobei als entscheidende Vorteile dabei die einfache, weniger Syntheseschritte umfassende Reaktion sowie eine dadurch bedingt einfache Produktisolierung zu nennen sind.

Das bedeutet in der Konsequenz auch einen geringeren Lösungsmittel- und Energieverbrauch.

Die Erfindung betrifft somit ein Verfahren zur Herstellung von 5-(4-[4-(5-Cyano-3-indolyl)-butyl]-1-piperazinyl)-benzofuran-2-carboxamid und/oder eines seiner physiologisch unbedenklichen Salze, wobei man eine Verbindung der Formel I 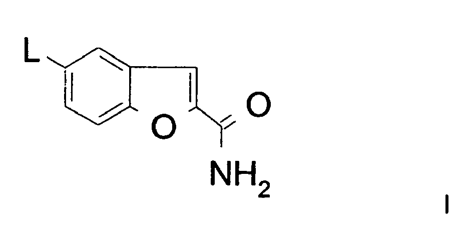 worin
L Cl, Br, I, SO₂F, SO₂CF₃, SO₂C₂F₅ bedeutet,
durch Übergangsmetall-katalysierte Kupplung mittels Pd-Komplexen mit 3-(4-Piperazin-1-yl-butyl)-indol-5-carbonitril umsetzt, und/oder dass man das gebildete 5-(4-[4-(5-Cyano-3-indolyl)-butyl]-1-piperazinyl)-benzofuran-2-carboxamid durch Behandeln mit einer Säure in eines seiner Säureadditionssalze umwandelt. (Verfahrensvariante a)) Bevorzugt wird als Rest L Brom verwendet.

Die vorher genannte Verfahrensvariante a) basiert somit auf der metallkatalysierten Kupplung eines Piperazins in Gegenwart eines Übergangsmetallkatalysators mit einem 5-Halogen-indol-Derivat, vorzugsweise einem 5-Brom-indol-Derivat und führt zum vorher erwähnten Endprodukt. Im Vergleich zu den, aus dem Stand der Technik, bekannten Verfahren, welche bis zu 10 Stufen umfassen können, ist diese Verfahrensvariante deutlich kürzer und somit auch kostengünstiger.

Als Übergangsmetall-Katalysatoren werden vorzugsweise Pd(0)-Komplexe wie Tris(dibenzylidenaceton)-dipalladium oder analoge Komplexe in Kombination mit Phosphor-Liganden wie z.B. P(t-Bu)₃ eingesetzt. Aber auch Pd²⁺-Derivate wie z.B. PdCl₂ oder Pd(OAc)₂ können als Palladium-Quelle verwendet werden.

Bekannt sind Verfahren zur Herstellung von 5-(1-Piperazinyl)-benzofuran-2-carboxamid z.B. aus WO 01/40219 (Merck Patent GmbH), wobei ein Übergangsmetallkatalysator verwendet wird. Für die metall-katalytische Kupplung werden aprotische Lösungsmittel wie Toluol, Xylol, THF oder andere Ether verwendet.

Als Basen eignen sich Alkalialkoholate, vorzugsweise Natrium-tert-butylat oder auch Alkalicarbonate.

Die Reaktionszeit liegt je nach den angewendeten Reaktionsbedingungen zwischen einigen Minuten und 7 Tagen, die Reaktionstemperatur zwischen 0 und 150° C, vorzugsweise zwischen 20° und 120° C.

Gegenstand der Erfindung ist auch ein Verfahren zur Herstellung von 5-(4-[4-(5-Cyano-3-indolyl)-butyl]-1-piperazinyl)-benzofuran-2-carboxamid und/oder eines seiner physiologisch unbedenklichen Salze, wobei man eine Verbindung der Formel II 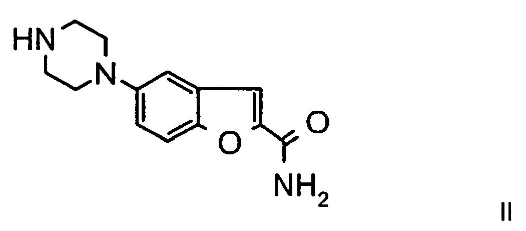 als Base oder HX-Salz (mit X = Cl, Br, Sulfat oder auch organische Gegenionen wie z.B. das Methansulfonsäure-Anion (CH₃ SO₃⁻)) mit 3-(4-Oxo-butyl)-1H-indol-5-carbonitril durch reduktive Aminierung umsetzt, und/oder wobei man das hierbei gebildete 5-(4-[4-(5-Cyano-3-indolyl)-butyl]-1-piperazinyl)-benzofuran-2-carboxamid durch Behandeln mit einer Säure in eines seiner Säureadditionssalze umwandelt (Verfahrensvariante b)).

Die reduktive Aminierung (Verfahrensvariante b)) von Aldehyden stellt eine verbreitete Amin-Synthese dar (siehe Baxter, E.W.; Reitz, A.B. Organic Reactions 2002, 59,1). Im vorliegenden Verfahren wird zunächst aus 3-(4-Hydroxy-butyl)-1 H-indol-5-carbonitril das Aldehyd 3-(4-Oxo-butyl)-1 H-indol-5-carbonitril gewonnen. Das Aldehyd wird im Folgeschritt mit einer Verbindung der Formel II unter Zusatz von Reduktionsmittel gekuppelt, wobei 5-(4-[4-(5-Cyano-3-indolyl)-butyl]-1-piperazinyl)-benzofuran-2-carboxamid als Base anfällt und mit laborüblichen Methoden isoliert werden kann oder aber die Base wird in Lösung oder als Feststoff nach Behandlung mit Säuren wie z.B. Salzsäure in das Mono-Hydrochlorid (Vilazodone) überführt.
Somit wird der Ersatz des Alkylierungsschrittes mit Reagenz 3-(4-Chlorbutyl)-indol-5-carbonitril, wie es in J. Med. Chem. (2004), 47(19), 4684-4692, Heinrich, T.; Boettcher, H.; Gericke, R.; Bartoszyk, G. D.; Anzali, S.; Seyfried, C. A.; Greiner, H. E.; van Amsterdam, C. und dort zitierter Literatur beschrieben ist, durch eine reduktive Aminierung mit (3-(4-Oxobutyl)-1 H-indol-5-carbonitril) möglich.

Als Lösungsmittel eignen sich Alkohole, vorzugsweise Methanol, aber auch Ether, Kohlenwasserstoffe oder andere Lösungsmittel, die die Edukte ausreichend lösen. Die Reaktionszeit liegt je nach den angewendeten Reaktionsbedingungen zwischen einigen Minuten und 7 Tagen, die Reaktionstemperatur zwischen 0 und 150° C, vorzugsweise zwischen 0° und 30° C.

In den nachfolgenden Beispielen bedeutet "laborübliche Aufarbeitung" dieses Prozedere: Man gibt, falls erforderlich, Wasser hinzu, stellt, falls erforderlich, je nach Konstitution des Endproduktes auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation.

Die Base von 5-(4-[4-(5-Cyano-3-indolyl)-butyl]-1-piperazinyl)-benzofuran-2-carboxamid kann mit einer Säure in das zugehörige Säureadditionssalz überführt werden, z.B. durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie HCl oder HBr, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon, Sulfon- oder Schwefelsäuren,

### Metall-katalytische Aminierung (Verfahrensvariante a))

### Beispiel 1:

Unter Schutzgas werden 80 mg Tris(dibenzylidenaceton)dipalladium und 65 mg Tris-tert.-butylphosphin bei 20 °C in 70 mL Diethylenglycoldimethylether unter Rühren eingetragen. Anschließend werden 1,5g 5-Brombenzofuran-2-carboxamid und 2,5g 3-(4-Piperazin-1-yl-butyl)-indol-5-carbonitril eingetragen. Bei der anschließenden Zugabe von 2,3g Natrium-tert-butylat bildet sich eine gelb-graue Suspension. Das Rektionsgemisch wird für 48 Std auf 120°C erhitzt, dann das Reaktionsgemisch auf RT (ca. 23°C) abgekühlt, mit laborüblichen Methoden aufgearbeitet und die Zielverbindung wahlweise als Base von Vilazodone oder nach Behandlung der gelösten Base mit wässriger Salzsäure als Mono-Hydrochlorid (= Vilazodone / 5-{4-[4-(5-Cyano-3-indolyl)-butyl]-1-piperazinyl}-benzofuran-2-carboxamid-Hydrochlorid) isoliert.

Die Identität der Zielverbindung wurde durch chromatographischen Vergleich mit Referenzmaterial belegt.

### Reduktive Aminierung (Verfahrensvariante b))

### Beispiel 2:

### Vorstufe 3-(4-Oxo-butyl)-1H-indol-5-carbonitril

18 g 3-(4-Hydroxy-butyl)-1 H-indol-5-carbonitril und 34 ml Triethylamin werden in 300 ml Dichlormethan gelöst und im Eis/ Methanol - Bad auf ca. 0 °C heruntergekühlt. Anschliessend wird eine Lösung von 39 g Schwefeltrioxid-Pyridinkomplex und 140 ml Dimethylsulfoxid bei 2 bis 5 °C zudosiert. Es wird noch ca. 20 min. bei 2 bis 3 °C nachgerührt, dann die Reaktionslösung in 2 Std. auf 22 -23 ° C (Raumtemperatur) erwärmt. Die Reaktionsmischung wird durch Zugabe von weiteren 200 ml Dichlormethan verdünnt und danach mit Wasser, 10%iger Zitronensäurelösung und 10 %iger Kochsalzlösung extrahiert. Die organische Phase wird im Vakuum zu einem öligen Rückstand aufkonzentriert, dann an Kieselgel mit einer Mischung aus Dichlormethan und MTB - Ether chromatographiert.

Ein vergleichendes H-NMR und MS bestätigen die Identität.

### Herstellung von 5-{4-[4-(5-Cyano-3-indolyl)-butyl]-1-piperazinyl}-benzofuran-2-carboxamid-Hydrochlorid

Bei 20 °C werden 1,7 g 5-(1-Piperazinyl)-benzofuran-2-carboxamid und 1,1 g Natriumcyanoborhydrid in 200 ml Methanol unter Rühren gelöst. Eine Lösung aus 2,4 g 3-(4-Oxo-butyl)-1 H-indol-5-carbonitril und 50 ml Methanol werden bei dieser Temperatur innerhalb von 15 min. zugegeben. Die Reaktionsmischung wird ca. 18 h bei 20°C gerührt, dann für 6 Std. auf 10 °C gekühlt. Das ausgefallene feste Produkt wird abgetrennt, mit Methanol und Wasser gewaschen und im Vakuum getrocknet. Der verbleibende Feststoff wird bei 20 °C unter Rühren Tetrahydrofuran gelöst und filtriert. Das Filtrat wird mit wässriger 1 N HCl versetzt. Die Reaktionsmischung wird bei 20 °C nachgerührt, dann das ausgefallene feste Produkt abfiltriert. Der Filterrückstand wird mit THF und Wasser gewaschen und im Vakuum thermisch getrocknet.

Der gewonnene Feststoff stellt nach chromatographischen Vergleich mit Referenzmaterial die Zielverbindung Vilazodone (5-{4-[4-(5-Cyano-3-indolyl)-butyl]-1-piperazinyl}-benzofuran-2-carboxamid-Hydrochlorid) dar.

## Patentansprüche

1. Verfahren zur Herstellung von 5-(4-[4-(5-Cyano-3-indolyl)-butyl]-1-piperazinyl)-benzofuran-2-carboxamid und/oder eines seiner physiologisch unbedenklichen Salze, **dadurch gekennzeichnet, dass** man eine Verbindung der Formel I 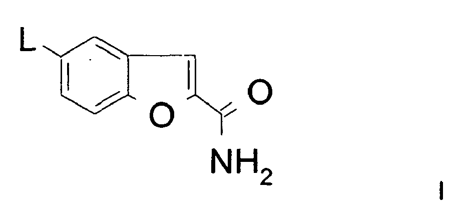 worin
L Cl, Br, I, SO₂F, SO₂CF₃, SO₂C₂F₅ bedeutet,
durch Übergangsmetall-katalysierte Kupplung mittels Pd-Komplexen mit 3-(4-Piperazin-1-yl-butyl)-indol-5-carbonitril umsetzt,
und/oder dass man das gebildete 5-(4-[4-(5-Cyano-3-indolyl)-butyl]-1-piperazinyl)-benzofuran-2-carboxamid durch Behandeln mit einer Säure in eines seiner Säureadditionssalze umwandelt.

2. Verfahren nach Anspruch 1, **dadurch gekennzeichnet, dass** in der Verbindung der Formel I L Br bedeutet.

3. Verfahren nach den Ansprüchen 1 und/oder 2, **dadurch gekennzeichnet, dass** als Übergangsmetall-Katalysator-System Tris(dibenzylidenaceton)-dipalladium oder analoge Pd(0)-Komplexe verwendet werden.

4. Verfahren zur Herstellung von 5-(4-[4-(5-Cyano-3-indolyl)-butyl]-1-piperazinyl)-benzofuran-2-carboxamid und/oder eines seiner physiologisch unbedenklichen Salze, **dadurch gekennzeichnet, dass** man eine Verbindung der Formel II 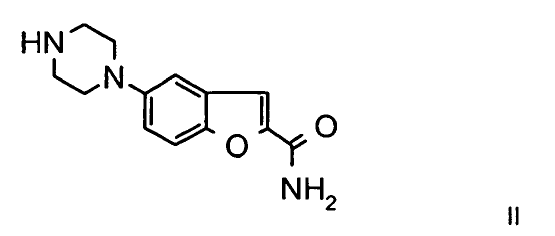 als Base oder HX-Salz (mit X = Cl, Br)
umsetzt mit 3-(4-Oxo-butyl)-1H-indol-5-carbonitril durch reduktive Aminierung,
und/oder dass man 5-(4-[4-(5-Cyano-3-indolyl)-butyl]-1-piperazinyl)-benzofuran-2-carboxamid durch Behandeln mit einer Säure in eines seiner Säureadditionssalze umwandelt.

## Claims

1. Process for the preparation of 5-(4-[4-(5-cyano-3-indolyl)butyl]-1-piperazinyl)benzofuran-2-carboxamide and/or one of its physiologically acceptable salts, **characterised in that** a compound of the formula I 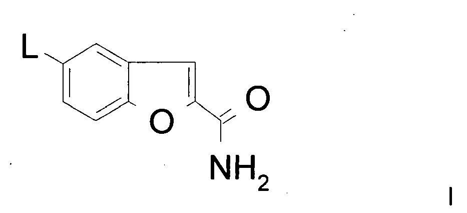 in which
L denotes Cl, Br, I, SO₂F, SO₂CF₃, SO₂C₂F₅,
is reacted with 3-(4-piperazin-1-ylbutyl)indole-5-carbonitrile by transition-metal-catalysed coupling by means of Pd complexes,
and/or **in that** the 5-(4-[4-(5-cyano-3-indolyl)butyl]-1-piperazinyl)benzofuran-2-carboxamide formed is converted into one of its acid-addition salts by treatment with an acid.

2. Process according to Claim 1, **characterised in that** L in the compound of the formula I denotes Br.

3. Process according to Claims 1 and/or 2, **characterised in that** the transition-metal catalyst system used is tris(dibenzylideneacetone)dipalladium or analogous Pd(0) complexes.

4. Process for the preparation of 5-(4-[4-(5-cyano-3-indolyl)butyl]-1-piperazinyl)benzofuran-2-carboxamide and/or one of its physiologically acceptable salts, **characterised in that** a compound of the formula II 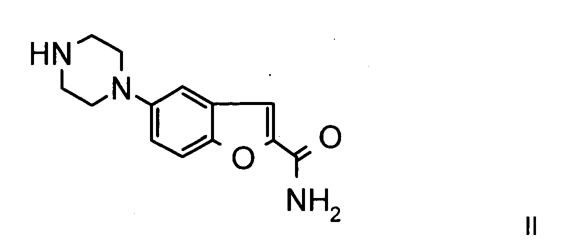 as the base or HX salt (where X = Cl, Br),
is reacted with 3-(4-oxobutyl)-1H-indole-5-carbonitrile by reductive amination,
and/or **in that** 5-(4-[4-(5-cyano-3-indolyl)butyl]-1-piperazinyl)benzofuran-2-carboxamide is converted into one of its acid-addition salts by treatment with an acid.

## Revendications

1. Procédé de préparation de 5-(4-[4-(5-cyano-3-indolyl)butyl]-1-pipérazinyl)-benzofuran-2-carboxamide et/ou de l'un de ses sels physiologiquement acceptables, **caractérisé en ce qu'**un composé de formule I 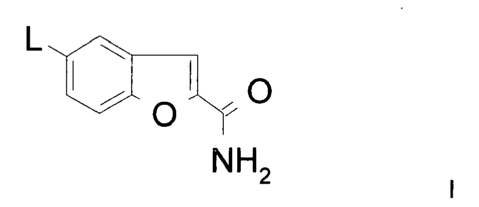 dans laquelle
L désigne Cl, Br, I, SO₂F, SO₂CF₃, SO₂C₂F₅,
est réagi avec du 3-(4-pipérazin-1-ylbutyl)indole-5-carbonitrile par couplage catalysé par un métal de transition à l'aide de complexes de Pd, et/ou **en ce que** le 5-(4-[4-(5-cyano-3-indolyl)butyl]-1-pipérazinyl)benzofuran-2-carboxamide formé est converti en l'un de ses sels d'addition d'acide par traitement par un acide.

2. Procédé selon la revendication 1, **caractérisé en ce que** L dans le composé de formule I désigne Br.

3. Procédé selon les revendications 1 et/ou 2, **caractérisé en ce que** le système de catalyseur de métal de transition utilisé est le tris(dibenzylidèneacétone)dipalladium ou des complexes de Pd(0) analogues.

4. Procédé de préparation de 5-(4-[4-(5-cyano-3-indolyl)butyl]-1-pipérazinyl)benzofuran-2-carboxamide et/ou de l'un de ses sels physiologiquement acceptables, **caractérisé en ce qu'**un composé de formule II 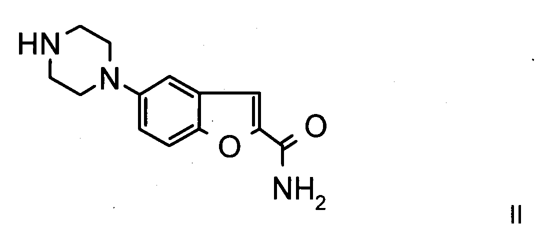 comme base ou sel de HX (où X = Cl, Br),
est réagi avec du 3-(4-oxobutyl)-1 H-indole-5-carbonitrile par amination réductrice,
et/ou **en ce que** le 5-(4-[4-(5-cyano-3-indolyl)butyl]-1-pipérazinyl)benzofuran-2-carboxamide est converti en l'un de ses sels d'addition d'acide par traitement par un acide.
